# EUROPEAN PATENT APPLICATION

(11) **EP 2 243 443 A1**
(43) Date of publication of application: **27.10.2010**
(21) Application number: 09158497.9
(22) Date of filing: 22.04.2009
(51) Int. Cl.: A61F 2/24, A61M 1/10

(54) **A stent comprising a tricuspid valve**

(71) Applicant: Van den Berg, Cornelis Johannes Maria, 6417 AX Heerlen (NL)
(72) Inventor: Van den Berg, Cornelis Johannes Maria, 6417 AX Heerlen (NL)
(74) Representative: Habets, Winand

(57) **Abstract**

The invention is in the field of devices intended for the treatment of heart failure of any origin with a substrate in the heart, in particular for the treatment of coronary diseases and/or arterial diseases More in particular, the invention provides an artificial valve that is suitable for permanently residing in an artery, more in particular the descending aorta, even more in particular the descending aorta just after branching of the left subclavian artery. A device according to the invention may be advantageously employed in the treatment of decompensatio cordis. A device according to the invention may be employed even if the arterial vessels are stiff.

## Description

### Field of the invention

The invention is in the field of devices intended for the treatment of heart failure of any origin with a substrate in the heart, in particular for the treatment of coronary diseases and/or arterial diseases More in particular, the invention provides an artificial valve that is suitable for permanently residing in an artery, more in particular the descending aorta, even more in particular the descending aorta just after branching of the left subclavian artery. A device according to the invention may be advantageously employed in the treatment of congestive heart failure or decompensatio cordis. A device according to the invention may be employed even if the arterial vessels are stiff.

### Background of the invention

Nowadays Artificial valves (mechanical or biological) are exclusively applied for replacing a malfunctioning heart valve. Such valves are usually inserted surgically. Recently, artificial valves have been described that may be implanted percutaneously.

Also, vascular valves for placement in smaller veins or arteries have been described. Vascular valves for placement in the descending aorta have also been described. Such valves have also been described mounted on stents, albeit for temporary placement in the patient's body to overcome a period of acute detoriation of decompensted heart failure. They are all catheter mounted, so they are prone to infections and thrombosis, can only reside a very limited period and make the patient bedridden and to be bound to an intensive cardiac care unit.

There remains a need for an artificial valve mounted on a low gradient stent that can withstand the forces applied on it by the blood stream in the aorta descendens. Such a device may then be applied to a mobile patient and remain in the patient's body for a lifetime.

### Summary of the invention

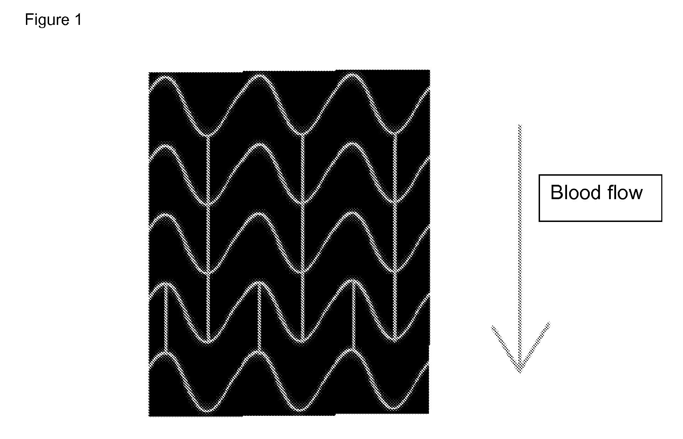

The device according to the invention comprises a low gradient valve mounted on a stent with an outer surface as depicted in figure 1. Such a device may be delivered percutaneously and transluminally and is non thrombogenic.This device provides structures that anchor the stent in the aorta descendens and prevent its delocalization by the blood flow in the aorta.

### Detailed description of the invention

This invention relates to a device that provides a mechanical support that may be used to treat congestive heart failure. Placement of the device at its intended location leaves the diseased structure of the existing heart untouched. It is a mechanical support, which does not need the addition of external energy and may be applied both in the chronic and acute setting. The device according to the invention may be applied under conditions of heart failure whereas a disfunctioning heart valve is not mandatory.

The device may be applied to the human or animal body by percutaneous and transluminal insertion. It is also possible to implant the device using a surgical method.

In the prior art, patients with severe chronic or acute heart failure are usually treated with drugs that may reduce the preload, reduce the afterload or strengthen the contraction force, without negatively influencing the coronary flow. Drug therapy with reduction of blood volume (ie diuretics, reducing pre-and afterload) and vasodilatantia (reducing afterload) are among the most commonly applied treatments.

Intravenous drugs such as catecholamines are usually supplied in order to influence the contraction force. Such treatment however is only effective for a limited period of exacerbation, for instance during cardiac asthma.

Mechanical intervention is often the ultimate but also the most effective treatment. For better understanding of the therapies which are used in the mechanics of the heart but also to see the uniqueness of the invention, it is useful to distinguish the type of intervention and of the surgery.

The mechanical interventions to change the mechanics of the heart can take place in the heart or in the large blood vessels outside the heart.

The mechanical functioning of the heart can be changed by electrical add-ons inserting a PM, a PM and ICD, biventricular pacing.

In the past, mechanical attempts to increase the contraction force through the so-called wrap heart, by which a prepared skeletal muscle was wrapped around the heart, required extensive preparation and operation and was ultimately not as effective as a therapy.

Mechanical intervention can take place in the structure of the heart so that congenitally defective structures are replaced by structures resembling the original structures as much as possible (eg ASD, VSD) or by removing obstructing structures (eg subaortale membrane, ASH, coronary stenoses) or by replacing defective dysfunctional structures by structures resembling the original structures as much as possible (eg AVR, MVR, PVR). In all these cases it is the aim to restore normal heart mechanics as much as possible by repairing, replacing or removing the defect.

By far most heart surgery in the world is carried out within this category of mechanical intervention.

Sometimes trying to restore the normal mechanics of the heart as close as possible is not the best way of intervention and an operation in the heart adding structures in the heart which support the failing heart but in which essentially normal blood flow and thus hemodynamics are changed can appear to be the best solution.

There are pumps that are relatively easily inserted, such as the Impella pump, the Pulse Cath or the ELS. Usually they are intended to support the heart during a high-risk percutaneous intervention (eg PVR, Main-stem dilation) . There are also pumps that can only be implanted by a major operation eg the well known cardiac assist devices

All these pumps operate on an external energy source and require a bedridden patiënt. Exceptionally such a device leaves the patiënt a limited mobility.

Finally, the mechanical functioning of the heart may be supported by an intervention outside the heart itself in the large vessels for the purpose to let the heart pump against a smaller resistance. The structure of the heart itself remains essentially unchanged.

If Hemoglobin content is high enough, bloodvein is the most simple form, reducing pre-and afterload by decrease in circulating volume but is still effective, even though the method is somewhat obsolete.

The use of the balloon pump is widespread and is still undergoing improvements. The coronary perfusion begin diastole by inflation of the balloon is increased while end-diastolic afterload is reduced by deflation. The IABP reduces especially afterload, but is only temporarily applicable, not in a serious sclerotic aorta and also requires energy administration from the outside.

The idea which is the basis for our invention, inserting a valve in the aorta descendens shortly after branching of the left subclavian artery for the treatment of chronic congestive heart failure is a unique solution for the problem to influence the defective mechanical functioning of the heart by an intervention outside the heart itself in the large vessels with the aim to relieve the heart by diminishing afterload.

In the run-up to replace the aortic valve with a mechanical valve the preservation of adequate coronary perfusion was twice a big problem. Therefore as a first step insertion of a valve in the aorta as close as possible to the aortic valve but behind the arteries of the head and the arms was chosen to study the effects of valve replacement. In both cases this application was limited to pure aortic insufficiency. This was reasonable from the viewpoint to replace the defect in the heart by approaching normal function as close as possible, thought in reconstructing defective structures in the heart

Precursors of the first surgically replaced aortic valve and the first percutaneously implanted aortic valve were valves both placed in the aorta descendens . These were the valve inserted in 1953 by Hufnagel and the percutaneous inserted valve described in 2000 by Boudjemline and Bonhoeffer.

According to Hufnagel and Boudjemline and Bonhoeffer it is not possible to implant the artificial valve between the coronaries and truncus brachiocephalicus because this would quickly lead to ischemia by compromising coronary flow.

It is therefore that these experiments fall within the scope of Changes to the heart itself by replacing the mechanical defect to restore the original function as much as possible and not in the scope of Changes outside the heart, in the large vessels.

Hufnagel has implanted in patients with severe Al events a ball in a cage high in the aorta descendens, he estimated that 75% of the cardiac output flows through the valve. It is estimated that some 4000 Hufnagel valves were implanted but only 55 are described well in the literature. The valve was in some cases 13 years in function. Hufnagel describes the effects of the valve in case of Al are decrease in afterload, an increase of cardiac output (up to 75%), decrease of the PAP, a decline in CTR, a reduction in NYHA class and increase exercise tolerance.

Boudjemline and Bonhoeffer have implanted a valve in the aorta descendens in lambs by percutaneous insertion after they made an Al artificially. Earlier they had successfully implanted pulmonary valves this way. They argued that minimum back flow is necessary for any valve for sustained performance. Boudjemline and Bonhoeffer describe an increase in CO and coronary flow and decrease in peripheral pressures.

US2006074483 describes a method of treatment and devices for the treatment of left ventricular failure. US3671979 describes a catheter mounted artificial heart valve for implanting in close proximity to a defective natural heart valve. US4056854 describes an aortic heart valve catheter. These are all catheter based mechanical techniques for treating the decompensated heart in the acute setting with temporary support, as far as we know these are the only three examples. The primary purpose of these three patented devices in the acute setting was to develop a cheaper solution than the IABP, with less monitoring needed. The entire expandable and impandable valves with mounting catheter remains in situ. The patient is bedridden and the valve must be removed after a short time.

Both Hufnagel and Boudjemline and Bonhoeffer state that implantation of a artificial valve between the coronary ostia and truncus brachiocephalicus quickly leads to ischemia by compromising coronary flow. The current valves most used for PAVR are the Core Valve Revalving (18F) and the Edwards Sapien valve. In addition, stent-valves in development are the Bonhoefer valve, valve Aortech, Panagua valve, 3-F valve, Palmaz-Baily valve, Direct Flow valve, AorTx cover and Sadra Lotus valve.

The invention is primarily aimed at intraluminal and percutaneous insertion of an organic 3-Strut self-expandable or balloon mounted artificial valve, with only flow possible in the blood flow direction, which is inserted in the aorta descendens, preferably positioned a few cm after the left subclavian artery and is called artificial valve in the aorta descendens (AVAD, Van den Berg valve). Preferably the valve can be delivered with a 24 18 or less French catheter.

In a preferred embodiment, the AVAD is a biological valve because this presents no resistance to blood flowing in the aorta (gradient over the valve 0 mm Hg).

The primary function of the valve is in reducing afterload downstream of the valve, where estimated 75% of Cardiac Output flows through. The compliance of the vascular system after this valve makes that during diastole the pressures are conducted only to the periphery and that the reflected wave of the first and higher order from the periphery are completely stopped in diastole.

This decrease in afterload has a positive effect so that the CO increases, the lower PAP will reduce the shortness of breath and the exercise tolerance increases.

Moreover, it is anticipated that positive effects will be augmented by increase of contractility due to less segmental dyssynchronicity within the left Ventricle.

It is also anticipated that about half of the beneficial effects that can be obtained by a balloonpump may be obtained by insertion of the AVAD.

Bicuspid or tricuspid artificial valves are both suitable for placement in the device. It should be noted however that incomplete closure of the bicuspid valve especially in aging of the valve, as usually some degeneration is seen, occurs less often with a tricuspid valve. The pull forces will in the tricuspidal case be more spread over the whole annulus of artificial valve and will thus be smaller per square mm of the aortic wall and thus beneficial in preventing stent migration.

The forces on the AVAD will only occur in retrograde direction during diastole, in the target population and is estimated to remain below 100 mm Hg, such as 75 or 50 mm Hg.. The fixation by means of an expandable stent in the aortic wall must resist these forces, being the preferred that the valve is placed at the upsteam end of the stent and that the stent is at least 5 cm long. The downstream side is in particular the fixation side.

In order to prevent overgrowth of the leaflets of the valve, the leaflets may comprise a drug eluting coating or equivalent means.

The meshes in the stent should be large enough to maintain unrestricted blood flow to branching arteries.

This invention is part of mechanical support techniques for a decompensated heart and is the only one which may be applied in the chronic setting even if the valves of the heart itself are intact.

The described invention is a simple outpatient applicable mechanical form of intervention that may be used for chronic heart failure in the mobile patient.

The AVAD provides the advantage of allowing a reduced risk of the procedure due to the percutaneous transluminal placement. This provides a reduced invasiveness and reduced complexity of insertion. It is a prerequisite for this technique that the aorta descendens after the left subclavian artery is intact over about seven or more centimeters.

A device according to the invention provides the advantage that the valve is able to sustain a backward pressure gradient of more than 100 mm Hg. Such is true even at an early stage, when the valve is still not covered with endothelium, without dislocation.

The device according to the invention may be placed in a smooth as well as a sclerotic aorta. If properly placed, i.e. at a few cm from the subclavian left artery and with a max length of 7 cm, the device will not provide any problems with prior arteries. In a device according to the invention, the chances of valve degeneration are minimal.and the chances of endothelial growth on the valve leaflets are also minimal. Moreover, there is no antegrade gradient over the device according to the invention. If the device according to the invention comprises Dacron, endothelialization is facilitated and any micro-embolies may be prevented.

A device according to the invention may be applied using a conventional balloon. The inserted valve mounted on the balloon has a minimum diameter to be able to pass the femoral artery without much risk of damage.

### Legends to the figures

Figure 1: Pattern of the wall of a stent according to the invention.

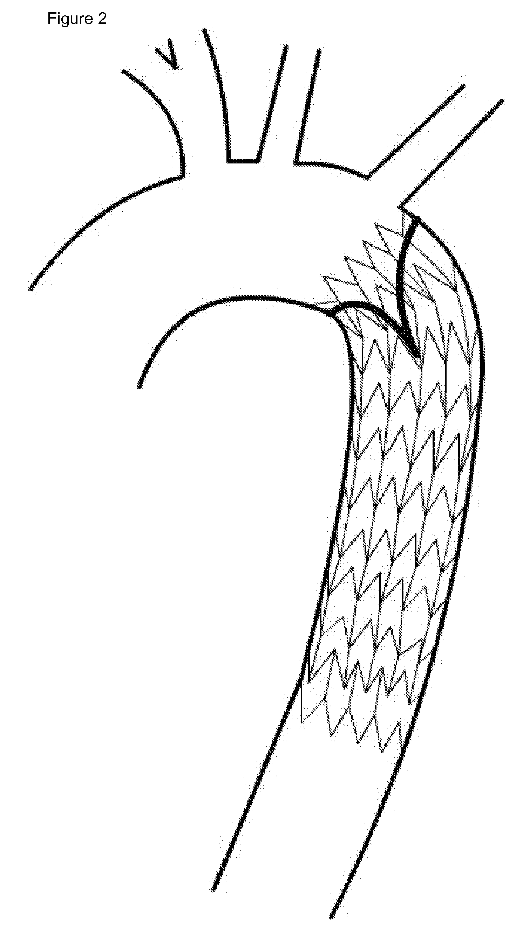

Figure 2: Stent with valve placed in the aorta descendens.

## Claims

1. A stent suitable for implantation in the aorta descendens comprising a tricuspid valve wherein the stent comprises a structure according to figure 1.
